# EUROPEAN PATENT SPECIFICATION

(11) **EP 2 804 533 B1**
(45) Date of publication and mention of the grant of the patent: **06.01.2016**
(21) Application number: 13700173.1
(22) Date of filing: 11.01.2013
(51) Int. Cl.: A61B 10/02

(54) **DEVICE FOR TAKING AT LEAST ONE SAMPLE OF TISSUE**
VORRICHTUNG ZUR ENTNAHME MINDESTENS EINER PROBE AUS EINEM GEWEBE
DISPOSITIF POUR LE PRÉLÈVEMENT D'AU MOINS UN ÉCHANTILLON DE TISSU

(30) Priority: 16.01.2012 EP 12290015
(43) Date of publication of application: 26.11.2014
(73) Proprietor: Coloplast A/S, 3050 Humblebæk (DK)
(72) Inventor: CALLEDE, David, F-24200 Sarlat la Caneda (FR); PIVARD, Laurent, F-01590 Dortan (FR); PINAUD, Denis, F-74550 Draillant (FR); TEPPE, Fabrice, F-01100 Oyonnax (FR); MOINE, Adrien, F-74500 Evian (FR)
(86) International application number: PCT/EP2013/050461
(87) International publication number: WO 2013/107696

(56) References cited:
- EP-A1- 2 407 112
- WO-A1-2007/103999
- WO-A1-2011/030693
- GB-A- 2 063 350
- US-A- 5 842 999

## Description

### TECHNICAL FIELD

The present invention relates to a device for taking at least one sample of soft tissue from an organ, said device comprising a body and a needle formed by a stylet and a cannula coaxial with said stylet, said device comprising a mechanism for arming the needle, designed for sequentially moving the cannula and then the stylet from a rest position wherein the stylet and the cannula are extended towards the outside of the body, to a shooting position wherein the stylet and the cannula are retracted towards the rear of the body, and a triggering mechanism designed to release the stylet then the cannula and to allow their displacement from the shooting position to the rest position, the cannula being coupled kinematically to a cannula slider comprising at least one retaining element for maintaining the cannula slider in a shooting position, the stylet being coupled kinematically to a stylet slider comprising at least one retaining element for maintaining the stylet slider in a shooting position and means for unlocking the cannula slider.

### BACKGROUND ART

Nowadays, there are several devices for taking samples of soft tissue, these devices being generally used to extract, in a minimally invasive way, a sample of an organ from a human or an animal for analysis purpose. This extraction operation is generally known as biopsy and the used device is known as a biopsy gun.

Such a sampling device comprises in particular a sampling needle formed by a cannula and a stylet, an arming mechanism placed on a body and a trigger also placed on the body of the device.

The arming mechanism is used to partially retract the needle towards the inside of the body of the device, the device is placed near the organ from which one wishes to take a sample, then the trigger is pressed so that the needle can penetrate into the organ. The needle being formed by a stylet and by a cannula, the stylet penetrates into the organ, the cannula then covers the stylet. This stylet comprises at least one notch receiving the tissue to be taken. When the cannula covers the stylet, the tissue sample is trapped in the notch and is cut. The unit is withdrawn so that the sample(s) arranged between the stylet and the cannula can be taken. An example of application of such a device is taking tissues of the prostate.

The arming of the needle is generally achieved in two phases, namely the arming of the cannula in a first phase and the arming of the stylet in a second phase.

During sampling of tissues, it is frequent that the person who carries out the sampling has only one free hand, the other hand being used to hold other medical devices such as for instance an echographic probe. In this case, it is important to be able to manipulate the sampling device with one single hand. The manipulation implies here the arming of the cannula, the arming of the stylet and the release of the shot allowing for the sample to be taken.

Among the existing devices, which enable a manipulation with one single hand, one of them is described in the US patent 7,153,275. This device is perfectly functional if it is manipulated in a correct way, i.e. in most cases. However, it happens that certain bad manipulations cause problems. In particular when the arming of the cannula or of the stylet has not been achieved correctly, the shot can be released in an unintentional way. This can cause problems because a shot can be released in particular before the device is correctly placed near the organ of which one wishes to take a sample.

US5842999 discloses an automated tissue sampling device which permits its needles to be cocked either simultaneously or sequentially and can be cocked with only one hand. The cocking mechanism is arranged so as to virtually eliminate the possibility that the needles will be cocked in the wrong sequence.The physician can readily confirm, either visually or tactilely, whether the device is uncocked, half cocked, or fully cocked.

Another problem that has been encountered with this kind of device is due to the fact that in case of incorrect manipulation the arming mechanism and the shooting mechanism may become totally jammed, thus rendering the device unusable.

An additional problem can occur when a traction force is applied to the cannula or to the stylet. This can occur particularly while the biopsy gun is put in place to take a sample. In that case, the elements that enable to maintain the stylet and/or the cannula in the armed position can be released from their support and cause an unintentional shot.

This invention proposes to realize a tissue sampling device which has the advantages of the devices of the prior art, i.e. it is possible to use this device with one hand. However, this device does not have the drawbacks of the systems of the prior art. Thus, even in the case of bad manipulation, the shot is not released in an unintentional way. Moreover, the device cannot be jammed as a result of a bad manipulation.

Another advantage of the device of the invention relates to the fact that even in case of a relatively large stress applied to the needle, for instance while this needle is put in place to be used to take a sample, an unintentional shot cannot be triggered. This feature is obtained without increasing the strength necessary for arming the gun.

Furthermore, and especially in implementations of the invention wherein the sampling device may be a single-use sampling device, particularly the risk of jamming of the needle and/or the cannula individually or in relation to each other is reduced or eliminated. This is at least partly because the sampling device, and particularly the movable parts thereof e.g. the needle and the cannula, is then assembled correctly during manufacture leaving no risks of a user putting the parts together in the wrong manner as could very well be the case with re-useable sampling devices. In addition, a single-use device is also significantly less prone to risks of contamination, e.g. by bacteria on a user's hands.

Moreover, as a single-use sampling device may enable production tolerances different from those of a re-useable sampling device, it is in most cases less costly to manufacture than such re-useable sampling devices. Thereby, the improved security mechanisms against unintentional firing of the sampling device according to the different implementations of the invention may be particularly, but not exclusively, suitable for single-use sampling devices in order to meet any potential risks due to such different production tolerances as mentioned above.

### DISCLOSURE OF THE INVENTION

The aim of the invention is reached by a sampling device as defined in the preamble and characterized in that at least one element among said retaining element of the cannula slider and said retaining element of the stylet slider comprises at least one hook, this hook comprising at least an elastic blade arranged for resting against a corresponding retaining device in a locking position and for being released from the corresponding retaining device in a shooting position, said elastic blade having at least one bending area around which this elastic blade is bent under the action of a stress, and said bending area is placed at the rear of said corresponding retaining device.

According to the present invention, the device for taking samples comprises a needle formed by a cannula and a stylet. The cannula is integral with a cannula slider and the stylet is integral with a stylet slider. These sliders comprise retaining means that enable the sliders to be maintained in such a position that the stylet and/or the cannula are armed i.e. they are in a position ready for a sampling shot. With this invention, the stronger the traction force is on the sliders or on the needle, the stronger the retaining force of the sliders will be.

Thus, the risk of releasing the sliders during the manipulation of the device and thus to produce an unintentional shot is very low.

Thanks to the invention, it is possible to ensure a high retention force of the sliders while having a relatively low arming force, or at least a force which is not increased compared to the devices of the prior art. According to a variant, the stiffness of the springs used for the shot can be increased, without creating an additional risk of releasing the sliders. The increase of the stiffness of the springs enables a higher shooting speed, which leads to a better sampling.

By virtue of the geometry of the device, the propulsion and retaining elements for the stylet and the cannula can be arranged symmetrically around a longitudinal axis materialized by the stylet. This ensures that there are few transversal forces. Such transversal forces have the effect of increasing the friction between the parts, of causing wear and of risks of rupture as well as of jamming. By suppressing these transversal forces, it is possible to use smaller springs as it is no longer necessary to fight against friction. The biopsy gun is thus easier to use since the arming is made easier. Moreover, the gun can be used more often since the jamming risk is reduced.

For a similar arming force, the gun can have a higher shootingspeed than the prior art guns since a larger part of the available energy is used for the shot and not for overcoming frictions.

Having symmetrical retaining elements for the stylet and the cannula ensures a greater security since at least two elements retain the stylet and the cannula. Moreover, in case of an asymmetrical retaining element, a force can act on the retaining element and possibly deform it and/or unhook it. This can lead to jamming, ruptures or an unintentional shot.

### BRIEF DESCRIPTION OF THE DRAWINGS

This invention and its advantages will be better understood with reference to the enclosed drawings and to the detailed description of a particular embodiment, in which:
- Figure 1 represents a device for taking samples according to this invention;
- Figure 2a shows a first embodiment of a detail of the device of the invention, in a first position;
- Figure 2b is a view similar to figure 2a, the sampling device being in a second position;
- Figure 3a represents a second embodiment of the device for taking samples according to the present invention;
- Figure 3b is a view similar to figure 3a, the device being in a second position;
- Figure 3c is a view similar to figure 3a, when a traction is applied to a part of the device;
- Figure 4 is a view of a variant of a detail of the device of the invention; and
- Figure 5 is a view of an additional variant of the device of the invention.

### BEST MODE FOR CARRYING OUT THE INVENTION

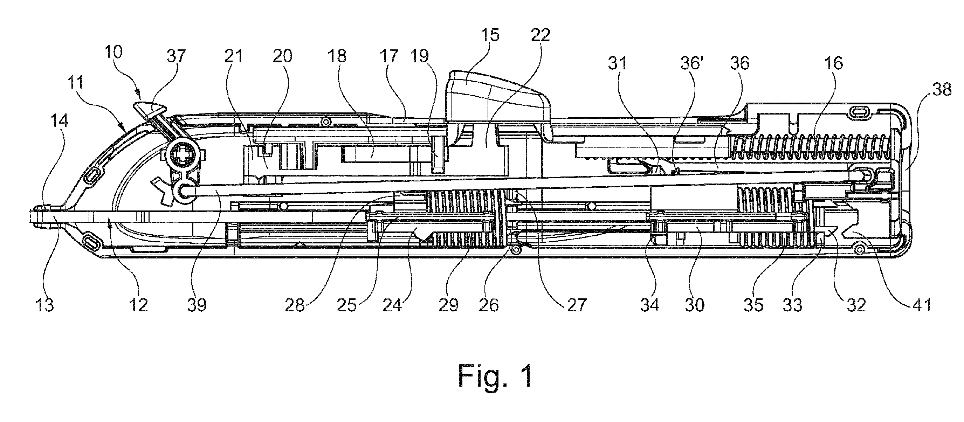

Figure 1 represents the device 10 for taking samples according to the invention almost in its entirety. This device essentially comprises a body 11 and a needle 12. The needle is formed by a stylet 13 and by a cannula 14. The stylet comprises a tip (not shown) allowing a penetration of the needle into the organ from which one wishes to take a sample. Furthermore, this stylet comprises at least one notch (not represented). In practice, the stylet 13 comprises a relatively long notch that enables a sample of great length to be taken. The cannula 14 slides around the stylet 13 and is used both to section the tissue into which the stylet has penetrated and to keep in place the tissues taken at the time of the extraction of the needle from the organ.

The body 11 essentially comprises an arming mechanism arranged to arm the needle 12 and a triggering device arranged to release a shot of the needle for the intended sampling. More particularly, the arming of the needle is carried out in two phases, namely a phase of arming the cannula 14 and a phase of arming the stylet 13.

The sampling is made by a shot of the needle. Such a shooting also comprises two phases, namely a displacement phase of the stylet 13 under the effect of a propelling power of the stylet, then a displacement phase of the cannula 14 under the effect of a propelling power of the cannula. Releasing a shot is achieved by liberating the displacement of the stylet. The displacement of the cannula is a consequence of the release of the stylet as it will be explained in detail below.

In practice, the mechanism for arming the cannula and the mechanism for arming the stylet use only one arming button 15 which acts differently depending on whether the arming of the cannula has already been carried out or not. This arming button cooperates with a return spring 16 of the arming button, this spring having the function to bring back the arming button 15 to the rest position i.e. towards the front of the body, when it is not manipulated.

The body of the device is formed by two parts which, once assembled, can comprise guidance grooves intended to ensure the displacement and the guidance of the parts. The body also comprises a slit 17 in which the arming button moves. It should be noted that other embodiments could be considered. For instance, the body can have one or more guidance surfaces or one or more rails arranged for cooperating with elements complementary to the sliders in order to ensure guidance. According to another variant, it is possible to provide no rotational guidance for the mobile element.

The arming button 15 cooperates with a platform 18. This platform can pivot around a platform axis 19 integral with the arming button. One of the ends of the platform, located near the front end of the sampling device i.e. the needle-end of the sampling device, comprises a widened zone 20, each end of this widened zone including a finger 21 whose function is described in detail below. The rear end of the platform comprises a pushing device 22 whose function is also described in detail below.

The platform 18 is connected to the arming button 15 by the platform axis 19 and by a return device (not represented) that can be in particular a spring or an elastic tab and which has the function of keeping this platform in a predefined position called a rest position.

The mechanism for arming the cannula 14 is intended to move the cannula into the shooting position. This cannula is coupled to a cannula slider 24. According to one advantageous embodiment, the cannula slider 24 comprises two fins 25 disposed in a plane containing also the cannula. These two fins 25 cooperate with two guide grooves realized in the body of the device so as to ensure an effective sliding motion of the cannula slider 24. This slider comprises, at its rear end, a retaining element 26 of the cannula slider. According to the invention, the retaining element is formed by at least one hook, ideally two hooks. Advantageously, these hooks are symmetrical and realized so as to have a certain flexibility, which allows for them to be hooked onto a retaining device 27 of the cannula slider and to be unhooked from this device by approaching the hooks to each other. It is also possible to use only one hook or several hooks arranged asymmetrically.

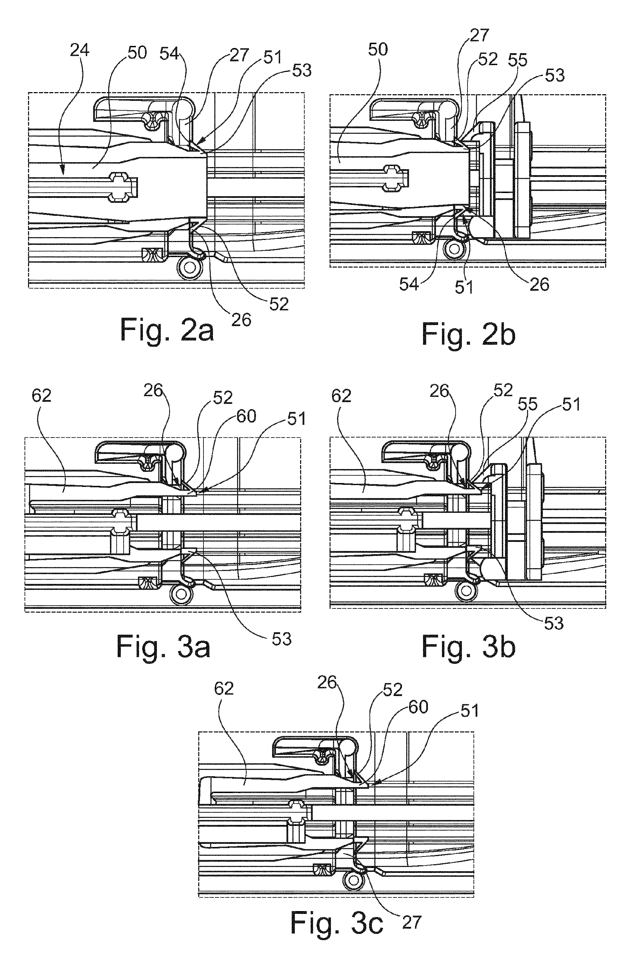

The retaining element 26 of the cannula slider is represented and described in more detail with reference to figures 2 to 5. In the embodiment illustrated in particular by figures 2a and 2b, the retaining element 26 is formed by a sleeve 50 in which end are mounted two hooks 51. These hooks comprise an elastic blade 52. The elastic blade can be pivotal with respect to the sleeve 50 for being able to rest on a retaining device 27 of the cannula slider, or on the contrary, for passing through this retaining device 27, in order to release the cannula slider 24. Both positions can be achieved thanks to the elasticity of elastic blades 52.

The elastic blades can pivot around a bending area 53, such bending area being positioned at the rear of the retaining element 27 of the cannula slider.

When a traction force is applied on the cannula slider 24, in the absence of pieces forcing the elastic blades 52 towards the centre, the blades are supported against the retaining element 27 of the cannula slider. Due to the fact that the bending area 53 of the elastic blades is placed at the rear of the retaining element 27, if a forward force is applied to the cannula slider or to the cannula itself, the elastic blades 52 will tend to undergo a deformation that will increase the retaining force of the slider. In this way, the more the forward stress is important on the slider, the more the retaining force applied by elastic blades will also be important.

The elastic blades illustrated by figures 2a and 2b comprise a shoulder 54. This shoulder is provided for resting against an edge of the retaining device 27 of the cannula slider, and thus for preventing the displacement of the elastic blades 52 towards the periphery of the device.

In figure 2a, the hooks 51 are placed on the retaining device 27 of the cannula slider in order to maintain this cannula slider in a locking position i.e. in a position in which the slider is locked against the corresponding retaining device. In figure 2b, a striker 55 is placed near the hooks 51. This striker is arranged for bringing the elastic blades 52 close together or for bringing them close to the centre of the retaining device and thus to unhook them from said retaining device 27. This releases the cannula slider which is therefore able to move to carry out a shot.

With reference to figures 3a, 3b and 3c, the hooks 51 are formed of an elastic tab 60, in addition to the elastic blade 52. The elastic tab 60 is a longitudinal tab that extends substantially along a longitudinal axis of the body of the sampling device 10. The elastic blade 52 is linked to the longitudinal elastic tab 60 by a bending area 53. This elastic blade 52 is arranged for resting against the retaining device 27 of the cannula slider. It can also be released from said retaining element when a sampling shot has to be carried out.

As in the embodiment described in reference to figures 2a and 2b, the elastic blade 52 comprises a bending area 53 positioned at the rear of the retaining device 27. As a result, the more the stress applied to the hooks is important, the more the retaining force will also be important.

In the embodiment shown in figures 3a to 3c, the hooks 51 also present a bending area 62 positioned in front of the retaining device 27. This allows the hooks to have a bigger flexibility. As a result, the force required for arming the cannula slider and thus for allowing the hooks 51 to pass through the retaining device 27 is lower than in the embodiment shown in figures 2a and 2b.

Figure 3a represents a position similar to figure 2a.

Figure 3b is a view similar to figure 2b. A striker 55 is represented near the hooks, this striker being responsible for separating the hooks from the retaining element and thus for releasing the cannula.

Figure 3c shows the position of the hooks when a forward stress is applied to the cannula slider or to the cannula itself. In this case, the elastic blade 52 is moved apart and a greater force is applied to the retaining device 27 of the cannula slider. This ensures a better bearing of the slider. This is possible since the hooks comprise a bending area 53 placed at the rear of the retaining device.

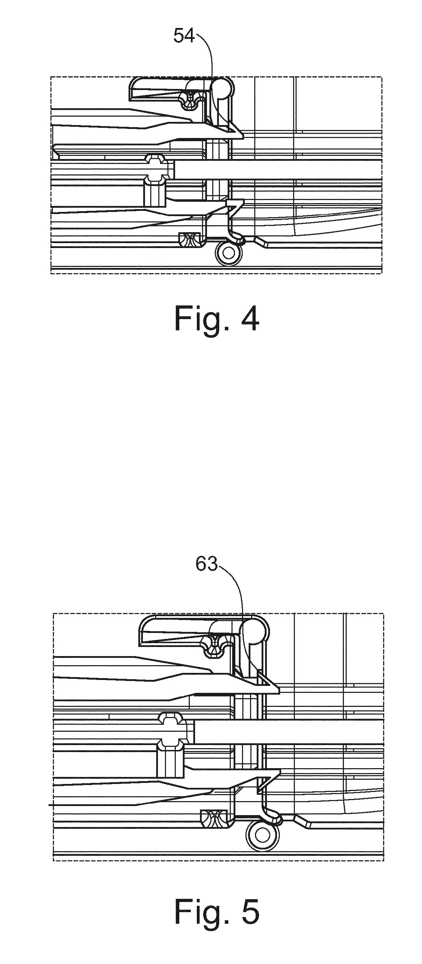

In the embodiments of figures 4 and 5, a shoulder is provided for limiting the stroke of the hooks. In the embodiment disclosed in figure 4, the shoulder 54 is identical to that represented in figures 2a and 2b. This shoulder is thus made on the hooks themselves. When the cannula is armed or in a locking position, the hooks are positioned against the retaining device 27 of the cannula slider and maintain this position until a striker releases them.

In the embodiment illustrated by figure 5, the shoulder is made on the retaining device. This shoulder 63 acts as a stop for limiting the stroke of the elastic blade 52. This stroke limitation also has the effect of limiting deformations applied to these elastic blades. This allows the use of smaller sized blades, which enables to reduce the force required for the arming.

The presence of a shoulder 54, 63, whether on the hooks or on the retaining device, ensures a good retention of the hooks on this retaining device and consequently, a good retention of the cannula slider.

Referring again to figure 1, the cannula slider 24 furthermore comprises a spur 28 cooperating with one of the fingers 21 of the platform. The cannula slider cooperates with a spring 29 for the propulsion of the cannula slider, which is arranged between the cannula slider 24 and the retaining device 27 of the cannula slider. This spring 29 is designed to supply the required force to propel the cannula slider towards the front of the body. The displacement of the cannula slider towards the back of the body effects the compression of this spring.

The mechanism for arming the stylet is intended for the displacement of the stylet into the shooting position, this displacement being achieved after the cannula 14 has been armed. To that effect, the stylet 13 is kinematically coupled to a stylet slider 30, which comprises a spur 31 near its front end and a retaining element 32 at its rear end. Like for the cannula slider, the retaining element 32 can be formed by two partially elastic hooks. It can also be formed only by one hook or by several hooks arranged symmetrically or asymmetrically.

The hooks 51 illustrated in figures 2 to 5 have been described with reference to the cannula slider 24 and to the corresponding retaining device 27. It is clear that similar hooks can be used for the stylet slider 30 that cooperates with a corresponding retaining device 33. As a result the above description made with reference to the hooks of the cannula sliders can be adapted in order to be applied to the stylet slider. The operation of the hooks as well as their benefits is the same, whether for the cannula slider or the stylet slider.

The retaining element 32 can be hooked on a retaining device 33 of the stylet slider and can be unhooked from this device by approaching the hooks to each other. The displacement of the hooks can be carried out by means of a striker.

Like for the cannula slider, the hooks of the stylet slider are sufficiently flexible to be able to be deformed one towards the other and sufficiently rigid to be able to be maintained on an adequate support.

The stylet slider 30 comprises, at its front end i.e. at the side of the cannula slider 24, means for unlocking 34 the cannula slider formed for instance by two inclined planes. These unlocking means comprise the striker 55 illustrated in figures 2b and 3b.

The stylet slider 30 cooperates with a spring 35 for the propulsion of the stylet slider, which is placed between the stylet slider 30 and the retaining device 33 of the stylet slider. This spring is designed to supply the required force to propel the stylet slider 30 towards the front of the body. The displacement of the stylet slider towards the back of the body effects the compression of this spring.

The device of the invention further comprises a security element 36 which can advantageously be formed by a security hook cooperating with a rear shoulder 36' of the stylet slider and with the spur 31 of this slider.

The device according to this invention further comprises a triggering device. According to an advantageous embodiment, this triggering device comprises two triggers 37, 38 connected together by a rod 39. One of the triggers 37 is placed in the front of the body, in front of the arming button 15 and the other trigger 38 is placed in the rear of the body. The rear trigger 38 is associated with a return spring of the trigger, designed to bring the trigger back in the original position after it has been pressed. This enables the user to easily access the triggering mechanism, whatever the position of the hand when using the device.

The rear trigger 38 comprises means for unlocking 41 the stylet slider formed by two elements arranged in inclined planes. These unlocking means may comprise a striker similar or identical to the striker 55 illustrated on figures 2b and 3b.

The sampling device according to this invention operates in the following way. Let us assume that the initial position is a position in which the cannula 14 and the stylet 13 are maximally extended towards the outside of the body 11 of the device. This position corresponds to the normal position of the device when it is not going to be used, i.e. rest position.

In a first phase, the arming of the cannula 14 is carried out. During this operation, the user actuates the arming button 15, making it slide towards the back of the device 10. The platform 18 being integral with the arming button 15, the displacement of the latter also draws the platform backwards. One of the fingers 21 of the platform 18 comes in contact with the spur 28 placed towards the front end of the cannula slider 24. The latter is thus displaced backwards, in opposition to the force of the spring 29 for the propulsion of the cannula slider. This movement is carried out until the retaining elements 26 of the cannula slider 24 enter into contact with the retaining device 27 of the cannula slider. This retaining device 27 is for instance a ring realized in the body of the device. The ring comprises a central hole in which the ends of the hooks of the cannula slider pass. These hooks lean on the back face of the ring and maintain the cannula slider 24 in opposition to the force of the propulsion spring of this cannula slider.

According to an advantageous embodiment, at the end of the stroke of the platform i.e. just before the retaining elements 26 of the cannula slider are maintained by the corresponding retaining device 27, the platform 18 comes in contact with the spur 31 of the stylet slider and displaces the latter slightly backwards. Following this displacement, the hook forming the security element 36 cooperates with the rear shoulder 36' of the stylet slider and retains this slider in this position by preventing it from moving forward.

The end of the stroke of the platform also has the effect of displacing the cannula slider 24 into a position such that the retaining element 26 of the cannula slider is maintained on the retaining device 27 of the cannula slider. The position achieved during this movement is a locking position.

When the stylet slider 30 is retained by the security hook, the unlocking means 34 being part of the stylet slider 30 or in other words, the means for unlocking the cannula slider, cannot move sufficiently forward to separate the hooks of the cannula slider from the retaining organs 27 of these hooks. In this way, if the arming of the cannula is not carried out beforehand the hooks of the cannula slider do not hook to the corresponding retaining device, which is immediately detected by the user who simply needs to restart the arming of this cannula. If the arming of the cannula has been carried out correctly, the hooks of the retaining device are maintained in place and the hook of the security element 36 cooperates with the stylet slider 30 so as to prevent from advancing beyond a predetermined position. In this way, an unintentional release of the shot is not possible.

When the arming of the cannula is terminated, the arming button 15 is released. It returns to its initial position towards the front of the device, under the effect of the return spring 16 of the arming button.

During the forward displacement of the platform 18, following the forward displacement of the arming button 15, a ramp (not shown) of the platform comes into contact with a plug (not shown) realized in the body. This ramp has the effect of rotating the platform 18 around the platform axis 19, against the force of the return device of the platform. It should be noted that according to the chosen practical realization, it is also possible to provide for the return device of the platform to be constrained before the arming of the cannula and to be liberated when the arming of the cannula is terminated.

For the arming of the stylet 13, the arming button 15 is displaced backwards again. However, the platform 18 is no longer in the initial position. Indeed, the latter has pivoted around the platform axis 19, as the ramp of the platform has been displaced by the support against the plug. By this rotation, the finger 21 of the platform does not, on one side,come into contact with the spur 28 of the cannula slider, and on the other side, the pushing device 22 of the platform leans against the spur 31 of the stylet slider. In a first phase, the pushing device 22 is placed next to the spur 31 while in a second phase, the pushing device 22 rests against the spur 31.

The stylet slider is thus displaced towards the back of the device, in opposition to the force of the propulsion spring 35 of the stylet slider, until the retaining elements 32 of the stylet slider are placed in the retaining device 33 of the stylet slider. This retaining device is similar to the retaining device 27 of the hooks of the cannula slider. It has thus advantageously an annular form with a hole into which the hooks of the stylet slider fit. This is shown by figures 2 to 5.

At this stage, the device is triggered out and ready for the shot. The device is stable in the sense that the cannula and stylet slider hooks are maintained against the corresponding retaining elements. The hook of the security element 36 is no longer in contact with the rear shoulder 36' of the stylet slider. The arming button 15 is released and returns to its initial position under the effect of the return spring of the arming button. The platform 18 also returns to its initial position.

If the arming of the stylet is not carried out correctly and the hooks of the cannula slider are not well maintained on the corresponding retaining device, the stylet slider moves in direction of the cannula slider. The security element 36 cooperating with the rear shoulder 36' of the stylet slider prevents the unlocking means 34 connected to this stylet slider (or means for unlocking of the cannula slider) from interacting with the retaining element 26 of the cannula slider. Thus, even in case of bad manipulation during the arming of the stylet, an unintentional shot cannot be released.

When the needle is armed, the sampling is started by a shot. This shot can be started by means of one of the triggers 37, 38. According to an advantageous embodiment, a security mechanism is provided for preventing a shot during an involuntary manipulation of one of the triggers and in particular of the front trigger. Before the release of the shot, it is necessary to laterally displace this front trigger 37 in relation to the body 11 in order to remove the security function of the mechanism. After the shot, it is necessary to laterally re-displace the front trigger 37 in order to reactivate the security function. This security function is manual in the sense that the user has the choice whether to activate the function by displacing the trigger or not.

To release the shot, it is necessary to press one of the triggers 37, 38, the front or the rear one. Actually, in the disclosed embodiment, the shot is always released by a displacement of the rear trigger 38. However, the front trigger and the rear trigger being linked by the rod 39, a pressure on the front trigger has as result to move the rear trigger forward under the pressure of the rod. Thus the mechanism can be used by pressing either the rear trigger or the front trigger.

When the rear trigger 38 is pressed, the unlocking means 41 being part of the rear trigger (or means for unlocking the stylet slider) comes into contact with the hooks of the stylet slider and displaces them towards each other. In this way, they are released from the retaining device 33 of the stylet slider. This slider 30 is propelled forward under the effect of the propulsion spring 35 of the stylet slider.

As the hook of the security element 36 is integral with the rear trigger 38, the fact of displacing this trigger forward also has the effect of displacing the security hook forward and upward. Thus the stylet slider 30 is no longer retained by this hook and can advance far enough so that the unlocking means 34 being part of this stylet slider, or the corresponding striker, come into contact with the hooks 26 of the cannula slider 24.

The means 34 for unlocking the cannula slider comes into contact with the hooks of the cannula slider, presses these hooks towards the centre and releases the retaining elements 27 of the cannula slider. The cannula slider 24 advances under the effect of the propulsion spring 29 of the cannula. This slider advances until it arrives at a stop realized in the body of the device. At this stage, the shot is terminated and the device can be withdrawn from the organ from which samples have been taken.

After the arming of the stylet, the platform 18 has returned to its rest position under the effect of the return device of the platform. After the shot, the pieces of the device return to their initial positions. The sample taken is confined between the stylet 13 and the cannula 14, in the notch provided for this purpose. This sample can be withdrawn by moving back the cannula, for instance by carrying out an arming movement as previously explained. When the arming of the cannula is terminated, it is possible to withdraw the sample without any risk because an unintentional shot is not possible. If a new sampling has to be carried out, the arming button is operated so as to arm the device totally and to make it ready for the shot. If it is not necessary to take a new sample, the arming is carried out as well and a blank shot is made.

The present invention has several advantages in comparison with the devices of the prior art. In particular, by the setup of the retaining elements 26, 32 of the stylet and cannula sliders, it is possible to provide at least two symmetrical hooks. The forces applied on these hooks to hold them by the retaining means as well as during their unhooking during a shot are symmetrical. On the one hand, this ensures that there is no flexing and/or twist on the needle, and on the other hand, this enables a safer support of the hooks.

The fact that the bending areas of the elastic blades are located at the rear of the retaining devices ensures a better security since the retaining force is increased when the stresses applied to these blades increase. This allows the use of elastic blades having a lower size than in the systems of prior art while at the same time maintaining similar retaining forces. Lower size elastic blades are advantageous since they need less force for being positioned on the retaining means. It is therefore possible to obtain, thanks to this, either a lower arming force, or to use springs having a larger stiffness for a same arming force. The larger stiffness of the springs enables to have a higher shoot speed, which enables to obtain samples of better quality.

According to an advantageous realization, the needle is off-center towards the bottom of the device 10. This enables the use of the device in an easier way with another apparatus as for example an echographic probe.

In case of incomplete movement during the arming of the cannula, the hooks of the cannula slider are simply not maintained on the corresponding retaining device and the locking position is not achieved. This has the advantage that an unintentional shot is not possible and that the arming of the stylet is not possible if the arming of the cannula is not done correctly.

The device according to the invention can be operated by one single hand since the arming of the cannula and the arming of the stylet use the same arming button.

By the symmetrical construction of the retaining elements of cannula and stylet sliders and by the position of the propulsion springs of these sliders, the stresses are divided symmetrically around the axis of the needle. Thus, the risks of jamming between the stylet and the cannula are minimized, which in some implementations of the invention enables the use of the device several times and thus allows for a greater number of samples to be taken.

The reduction of the jamming risk also allows for the reduction of the force of the propulsion springs while maintaining a high displacement speed for the sliders. This is advantageous for the user because a smaller force is necessary for arming the device. The manipulation with a single hand is easier in this way.

## Claims

1. Sampling device (10) for taking at least one sample of soft tissue from an organ, said device comprising a body (11) and a needle (12) formed by a stylet (13) and a cannula (14) coaxial with said stylet (13), said device comprising a mechanism for arming the needle (12), designed for sequentially moving the cannula (14) and then the stylet (13) from a rest position wherein the stylet (13) and the cannula (14) are extended towards the outside of the body (11), to a shooting position wherein the stylet (13) and the cannula (14) are retracted towards the rear of the body (11), and a triggering mechanism designed to release the stylet (13) then the cannula (14) and to allow their displacement from the shooting position to the rest position, the cannula (14) being coupled kinematically to a cannula slider (24) comprising at least one retaining element (26) for retaining the cannula slider (24) in a shooting position, the stylet (13) being coupled kinematically to a stylet slider (30) comprising at least one retaining element (32) for retaining the stylet slider (30) in a shooting position and means for unlocking the cannula slider (24), wherein the at least one element among said retaining element (26) of the cannula slider (24) and said retaining element (32) of the stylet slider (30) comprises at least one hook (51), **characterized in** the hook comprising;
at least an elastic blade (52) arranged for resting against a corresponding retaining device (27, 33) in a locking position and for being released from the corresponding retaining device (27, 33) in a shooting position,
said elastic blade (52) having at least one bending area (53) around which this elastic blade (52) is bent under the action of a stress, and said bending area (53) is placed at the rear of said corresponding retaining device (27, 33).

2. Sampling device according to claim 1, **characterized in that** the retaining element (26) of the cannula slider (24) and the retaining element (32) of the stylet slider (30) comprise an elastic blade (52).

3. Sampling device according to claim 1, **characterized in that** at least one element among said retaining element of the cannula slider (26) and said retaining element of the stylet slider (32) comprises a sleeve (50), this sleeve receiving said elastic blade (52).

4. Sampling device according to claim 3, **characterized in that** said sleeve (50) comprises a plurality of elastic blades (52) symmetrically located around a longitudinal symmetry axis.

5. Sampling device according to claim 1, **characterized in that** the hook (51) comprises an elastic tab (60) essentially placed along a longitudinal axis colinear to the needle and said elastic blade (52) is connected to this longitudinal tab by said bending area.

6. Sampling device according to claim 1, **characterized in that** the elastic blade (52) comprises a shoulder (54) arranged to be supported against said corresponding retaining device (27, 33) when the elastic blade (52) is in a locking position.

7. Sampling device according to claim 1, **characterized in that** at least one element among said retaining element (26) of the cannula slider (24) and said retaining element (32) of the stylet slider (30) comprises a shoulder (63) against which said elastic blade (52) is supported when being in locking position.

## Patentansprüche

1. Vorrichtung (10) zur Entnahme mindestens einer Gewebeprobe aus einem Organ, wobei die Vorrichtung einen Körper (11) und eine Nadel (12), geformt von einem Mandrin (13) und einer mit dem Mandrin (13) koaxialen Kanüle (14), umfasst, wobei die Vorrichtung einen Mechanismus zum Bereitmachen der Nadel (12) umfasst, der ausgelegt ist, die Kanüle (14) und dann den Mandrin (13) aus einer Ruhestellung, in welcher der Mandrin (13) und die Kanüle (14) zur Außenseite des Körpers (11) vorgeschoben werden, in eine Schussstellung, in welcher der Mandrin (13) und die Kanüle (14) zur Rückseite des Körpers (11) zurückgezogen sind, sequentiell zu bewegen, und einen Auslösemechanismus, der ausgelegt ist, den Mandrin (13) und dann die Kanüle (14) freizugeben und deren Verschiebung aus der Schussstellung in die Ruhestellung zu gestatten, wobei die Kanüle (14) kinematisch mit einem Kanülenschieber (24) verbunden ist, der zumindest ein Halteelement (26) zum Halten des Kanülenschiebers (24) in einer Schussstellung umfasst, wobei der Mandrin (13) kinematisch mit einem Mandrinschieber (30) verbunden ist, der zumindest ein Halteelement (32) zum Halten des Mandrinschiebers (30) in einer Schussstellung und Mittel zum Entriegeln des Kanülenschiebers (24) umfasst, worin das zumindest eine Element unter dem Halteelement (26) des Kanülenschiebers (24) und dem Halteelement (32) des Mandrinschiebers (30) zumindest einen Haken (51) umfasst, **dadurch gekennzeichnet, dass** der Haken Folgendes umfasst:
zumindest eine elastische Klinge (52), die ausgelegt ist, an einer entsprechenden Haltevorrichtung (27, 33) in einer Verriegelungsstellung anzuliegen und von der entsprechenden Haltevorrichtung (27, 33) in einer Schussstellung freigegeben zu werden,
wobei die elastische Klinge (52) zumindest einen Biegebereich (53) aufweist, um den diese elastische Klinge (52) unter der Wirkung einer Spannung gebogen wird, und wobei der Biegebereich (53) auf der Rückseite der entsprechenden Haltevorrichtung (27, 33) platziert ist.

2. Vorrichtung zur Entnahme einer Probe nach Anspruch 1, **dadurch gekennzeichnet, dass** das Halteelement (26) des Kanülenschiebers (24) und das Halteelement (32) des Mandrinschiebers (30) eine elastische Klinge (52) umfassen.

3. Vorrichtung zur Entnahme einer Probe nach Anspruch 1, **dadurch gekennzeichnet, dass** zumindest ein Element unter dem Halteelement des Kanülenschiebers (26) und dem Halteelement des Mandrinschiebers (32) eine Hülse (50) umfasst, wobei diese Hülse die elastische Klinge (52) aufnimmt.

4. Vorrichtung zur Entnahme einer Probe nach Anspruch 3, **dadurch gekennzeichnet, dass** die Hülse (50) eine Vielzahl von elastischen Klingen (52) umfasst, die symmetrisch um eine Längssymmetrieachse angeordnet sind.

5. Vorrichtung zur Entnahme einer Probe nach Anspruch 1, **dadurch gekennzeichnet, dass** der Haken (51) eine elastische Lasche (60) umfasst, die im Wesentlichen entlang einer Längsachse platziert ist, kolinear zu der Nadel, und die elastische Klinge (52) über den Biegebereich mit dieser langgestreckten Lasche verbunden ist.

6. Vorrichtung zur Entnahme einer Probe nach Anspruch 1, **dadurch gekennzeichnet, dass** die elastische Klinge (52) eine Schulter (54) umfasst, die ausgelegt ist, gegen die entsprechende Haltevorrichtung (27, 33) gestützt zu werden, wenn sich die elastische Klinge (52) in einer Verriegelungsstellung befindet.

7. Vorrichtung zur Entnahme einer Probe nach Anspruch 1, **dadurch gekennzeichnet, dass** zumindest ein Element unter dem Halteelement (26) des Kanülenschiebers (24) und dem Halteelement (32) des Mandrinschiebers (30) eine Schulter (63) umfasst, gegen welche die elastische Klinge (52) gestützt wird, wenn sie sich in der Verriegelungsstellung befindet.

## Revendications

1. Dispositif (10) d'échantillonnage pour le prélèvement d'au moins un échantillon de tissu mou sur un organe, ledit dispositif comportant un corps (11) et une aiguille (12) formée par un stylet (13) et une canule (14) coaxiale audit stylet (13), ledit dispositif comportant un mécanisme destiné à armer l'aiguille (12), conçu pour déplacer séquentiellement la canule (14) puis le stylet (13) d'une position de repos dans laquelle le stylet (13) et la canule (14) sont étendus vers l'extérieur du corps (11), à une position de tir dans laquelle le stylet (13) et la canule (14) sont rétractés vers l'arrière du corps (11), et un mécanisme de déclenchement conçu pour libérer le stylet (13) puis la canule (14) et pour permettre leur déplacement de la position de tir à la position de repos, la canule (14) étant accouplée cinématiquement à un coulisseau (24) de canule comportant au moins un élément (26) de retenue destiné à maintenir le coulisseau (24) de canule dans une position de tir, le stylet (13) étant accouplé cinématiquement à un coulisseau (30) de stylet comportant au moins un élément (32) de retenue destiné à maintenir le coulisseau (30) de stylet dans une position de tir et des moyens destinés à déverrouiller le coulisseau (24) de canule, ledit au moins un élément parmi ledit élément (26) de retenue du coulisseau (24) de canule et ledit élément (32) de retenue du coulisseau (30) de stylet comportant au moins un crochet (51), **caractérisé en ce que** le crochet comporte ,
au moins une lame (52) élastique agencée pour reposer contre un dispositif (27, 33) de retenue correspondant dans une position de verrouillage et pour être libérée du dispositif (27, 33) de retenue correspondant dans une position de tir,
ladite lame (52) élastique présentant au moins une zone (53) de courbure autour de laquelle cette lame (52) élastique se courbe sous l'action d'une contrainte, et ladite zone (53) de courbure est placée à l'arrière dudit dispositif (27, 33) de retenue correspondant.

2. Dispositif d'échantillonnage selon la revendication 1, **caractérisé en ce que** l'élément (26) de retenue du coulisseau (24) de canule et l'élément (32) de retenue du coulisseau (30) de stylet comportent une lame (52) élastique.

3. Dispositif d'échantillonnage selon la revendication 1, **caractérisé en ce qu'**au moins un élément parmi ledit élément de retenue du coulisseau (26) de canule et ledit élément de retenue du coulisseau (32) de stylet comporte un manchon (50), ce manchon recevant ladite lame (52) élastique.

4. Dispositif d'échantillonnage selon la revendication 3, **caractérisé en ce que** ledit manchon (50) comporte une pluralité de lames (52) élastiques situées symétriquement autour d'un axe de symétrie longitudinal.

5. Dispositif d'échantillonnage selon la revendication 1, **caractérisé en ce que** le crochet (51) comporte une patte (60) élastique placée essentiellement le long d'un axe longitudinal colinéaire à l'aiguille et ladite lame (52) élastique est reliée à cette patte longitudinale par ladite zone de courbure.

6. Dispositif d'échantillonnage selon la revendication 1, **caractérisé en ce que** la lame (52) élastique comporte comporte un épaulement (54) agencé pour venir s'appuyer contre ledit dispositif (27, 33) de retenue correspondant lorsque la lame (52) élastique est dans une position de verrouillage.

7. Dispositif d'échantillonnage selon la revendication 1, **caractérisé en ce qu'**au moins un élément parmi ledit élément (26) de retenue du coulisseau (24) de canule et ledit élément (32) de retenue du coulisseau (30) de stylet comporte un épaulement (63) contre lequel ladite lame (52) élastique vient s'appuyer lorsqu'elle est dans la position de verrouillage.
